# NEUE EUROPÄISCHE PATENTSCHRIFT

(11) **EP 1 372 575 B2**
(45) Veröffentlichungstag und Bekanntmachung des Hinweises auf die Entscheidung über den Einspruch: **26.05.2010**
(45) Hinweis auf die Patenterteilung: 23.11.2005
(21) Anmeldenummer: 02712962.6
(22) Anmeldetag: 28.03.2002
(51) Int. Cl.: A61K 6/10, C08K 5/01, C08L 83/04

(54) **DENTALE ABFORMMASSEN AUF SILIKONBASIS**
SILICON-BASED DENTAL IMPRESSION COMPOUNDS
MATIERES POUR EMPREINTES DENTAIRES A BASE DE SILICONE

(30) Priorität: 30.03.2001 DE 10116223
(43) Veröffentlichungstag der Anmeldung: 02.01.2004
(73) Patentinhaber: 3M ESPE AG, 82229 Seefeld (DE)
(72) Erfinder: ZECH, Joachim, 86916 Kaufering (DE); FETZ, Johann, 89464 Windach (DE); WANEK, Erich, 86916 Kaufering (DE); WAGNER, Ingo, 82237 Wörthsee (DE)
(86) Internationale Anmeldenummer: PCT/EP2002/003527
(87) Internationale Veröffentlichungsnummer: WO 2002/078647

(56) Entgegenhaltungen:
- EP-A- 0 152 887
- EP-A- 0 158 141
- EP-A- 0 166 107
- EP-A- 0 219 660
- EP-A- 0 891 763
- WO-A-00/59453
- DE-A1- 3 406 233
- US-A- 4 879 339

## Beschreibung

Die vorliegende Erfindung betrifft dentale Abformmassen auf der Basis additionsvernetzender Silikone sowie ein Verfahren zum automatischen Mischen dentaler Abformmassen auf Silikonbasis. Dabei zeichnen sich die Abformmassen durch eine nach ISO 4823 bestimmte Putty-Konsistenz im gemischten Zustand aus und bieten einen spürbar hohen Widerstand beim Einsetzen des mit Abformmasse gefüllten Abformlöffels in den Mund des Patienten.

In der zahnärztlichen Praxis werden häufig Silikon-Pasten zur Herstellung genauer Abformungen von Zähnen eingesetzt. Es handelt sich dabei um kaltvulkanisierende Zweikomponenten-Silikonkautschuksysteme, bei denen zwei Pasten vermengt werden, die dann nach einigen Minuten bei Raumtemperatur vernetzen. Derartige Silikon-Pasten können unterschieden werden in kondensationsvernetzende und additionsvernetzende Silikonabformmassen.

Additionsvernetzende Silikonabformmassen weisen im Gegensatz zu den kondensationsvernetzenden eine wesentlich geringere Schrumpfung auf und führen daher zu präziseren und besser lagerfähigen Abdrücken, die beliebig oft mit Gipssuspension zur Erstellung eines exakten Gipsmodells der Situation im Mund des Patienten ausgegossen werden können. Additionsvernetzende Silikonabformmassen sind daher in der Praxis bevorzugt.

Solche Abformmassen bestehen in der Regel aus zwei Komponenten, einer Basispaste, die Silikone, Füllstoff und Vernetzer enthält, sowie einer Katalysatorpaste, die Silikone, Füllstoff und Katalysator enthält. Die Aushärtung der Materialien erfolgt nach dem Mischen in genau definierten Volumenverhältnissen.

Die Mischung der Pasten erfolgt in der Regel manuell oder durch das Ausdrücken aus Doppelkammerkartuschen, wobei die Pasten durch ein Mischrohr gefördert werden, das einen statischen Mischer enthält. Damit sind aber nur relativ kleine Pastenmengen in kurzer Zeit mischbar. Da das Ausdrücken aus der Doppelkammerkartusche durch einen mit Handkraft betriebenen Dispenser vorgenommen wird, sind Pasten mit sehr hoher Viskosität nur sehr schwer mischbar, da die Handkraft begrenzt ist und durch den statischen Mischer im Mischrohr ein sehr hoher Widerstand gegen das Ausdrücken aufgebaut wird.

Durch die Entwicklung automatischer motorbetriebener Misch- und Dosiersysteme für zweikomponentige Abformmassen, die über eine automatische Förder- und Mischeinheit verfügen, wie sie in der US 5,286,105 beschrieben ist, wird die Notwendigkeit eines manuellen Mischens von Basis- und Katalysatorpasten hinfällig. Das Resultat ist ein völlig homogenes Produkt, das frei von Blasen ist.

Besonders häufig wird in der Praxis eine zweiphasige Abformmethode angewandt, bei der zunächst mit einer standfesten Abformmasse hoher Viskosität eine erste Abformung im Mund des Patienten vorgenommen wird. Diese wird nach der Aushärtung in Form eines vernetzten Gummis aus dem Mund des Patienten entfernt. Diese negative Form der entsprechenden Kiefersituation wird danach noch durch den Einsatz einer leichtfließenden, hochpräzisen, niedrigviskosen Abformmasse korrigiert und präzisiert und dann mit einem wässrigen Gipsbrei ausgegossen, der im erhärteten Zustand als Gipsmodell die Kiefersituation wiedergibt.

Additionsvernetzende Silikonabformmassen können in einer Reihe von verschiedenen Abformtechniken und -methoden angewendet werden, für die es jeweils unterschiedliche optimale Viskositäten bzw. Konsistenzen dieser Abformmassen im Markt mit dazu angepassten Abbinde- und Verarbeitungszeiten der jeweiligen Katalysatorpasten gibt.

In der ISO 4823 werden hierfür vier verschiedene Konsistenzen von Abformmassen definiert, nämlich eine leichtfließende (Typ 3), eine mittelfließende (Typ 2), eine schwerfließende (Typ 1) und eine knetbare (Typ 0).

Bei der Anfertigung der ersten Abformung ist für den Zahnarzt eine Konsistenz wünschenswert, die standfest ist und die beim Einsetzen des mit Abformmasse gefüllten Abformlöffels in den Mund des Patienten einen spürbaren Widerstand entgegensetzt. Dieser Widerstand führt dazu, dass der Prozess des Einsetzens gebremst wird, so dass ein gewisser Kraftaufwand vom Zahnarzt zu leisten ist. Dies führt dazu, dass die Gefahr des sogenannten Durchdrückens des Abformlöffels erheblich reduziert wird. Unter "Durchdrücken" ist die unerwünschte Situation zu verstehen, bei der der mit Abformmasse gefüllte Abformlöffel versehentlich zu weit in vertikaler Richtung auf den Oberkiefer bzw. Unterkiefer des Patienten hingeführt wird, so dass es zum Kontakt der Zahnoberflächen mit dem Boden des Abformlöffels kommt. An diesen Stellen ist die Oberfläche der betroffenen Zähne nicht mehr gänzlich von Abformmasse umgeben, weshalb es zu einem Informationsverlust in der Negativform der Abformung kommt. Abformmassen zur Anfertigung einer derartigen Erstabformung sollten daher einen hohen Widerstand beim Einsetzen des Löffels aufweisen, um diesen unerwünschten Effekt dadurch zu vermeiden, dass das Einsetzen eine gewisse Kraft erfordert, so dass der Löffel nicht zu schnell eingeführt werden kann und der Zahnarzt beim Einsetzen auch immer eine Kontrolle über die Vertikalbewegung im Mund des Patienten hat.

Eine Möglichkeit, Abformmassen mit einem Einbringwiderstand bereitzustellen, besteht darin, den Pasten eine hohe Viskosität zu verleihen. Solch hohe Viskositäten sind beispielsweise in sogenannten Knetmassen oder Puttymassen realisiert. Diese Silikonabformmassen werden in zweikomponentiger Form angeboten, bei der man die Basis- und die Katalysatorpaste mit Dosierlöffeln aus entsprechend gekennzeichneten Behältern entnimmt und die möglichst gleich großen Klumpen mit den Händen zu einer homogenen Masse verknetet. Um eine Knetbarkeit zu ermöglichen, können derartigen Pasten beispielsweise Isoparaffin oder Paraffin- oder Mikrowachs bzw. Paraffinöl oder mit Paraffinöl beschichtete Füllstoffe zugesetzt sein. Dadurch wird eine Klebrigkeit an den Fingern vermieden. Derartige Materialien werden beispielsweise in der EP-A 0 219 660, der EP-A 0 166 107 A1, der EP-A 0 158 141 und in der EP-A 0 152 887 beschrieben. Die nichtklebrige Konsistenz führt auch dazu, dass das gemischte Material im Abformlöffel noch geformt werden kann, um an gewünschte Stellen eine größere Pastenmenge schieben zu können.

Die US 4, 879 339 beschreibt eine lagerstabile Si-Abformmasse, die sich durch einen bestimmten Gehalt an aliphatischen Verbindungen (E), die mittels Antioxidantien (F) stabilisiert werden, auszeichnet. Bei dem Bestandteil (A) handelt es sich um ein Organopolysiloxan mit einer Viskosität von mindestens 50 centipoise bei 25°C. In Beispiel 1 wird eine Mischung zweier Dimethylpolysiloxane offenbart, wobei das eine eine Viskosität von 2500 centipoise aufweist und das andere eine Viskosität von etwa 1.000.000 centipoise.

Die WO 00/59453 offenbart eine Abformmasse auf Silikonbasis mit verbessertem Standvermögen.

Ein händisches Verkneten von Basis- und Katalysatorpaste ist bei diesen Massen erforderlich, da sie aufgrund ihrer hohen Viskosität mit einem üblichen Misch-Spatel auf einem Mischblock kaum gemischt werden können und weil eine automatische Mischung und Pastenförderung in einer Doppelkammerkartusche mit einem statischen Mischrohr nicht möglich ist.

Auch eine Förderung und Mischung in einem automatischen motorbetriebenen Misch- und Dosiersystem führt aufgrund zu hoher auftretender Kräfte zu einem Versagen des Gerätes. Dies äußert sich beispielsweise in einem unbefriedigenden bzw. inhomogenen Mischergebnis, einem starken Temperaturanstieg in der Mischkanüle, daraus resultierender, bereits in der Mischkanüle einsetzender Abbindereaktion und in einem vorzeitigen Verschleiß der Kupplung eines solchen Gerätes. Dennoch wäre es wünschenswert, wenn Abformmassen mit putty-ähnlichem Charakter sich auch automatisch mischen lassen könnten.

Um dies zu erreichen, müsste beispielsweise das Mischsystem entsprechend abgewandelt werden, sei es durch Erhöhung der Förderkraft oder entsprechender Änderungen der Düsengeometrie, durch die die zu mischende Paste gepresst werden muss. Es müßten daher die bestehenden Geräte durch nur aufwendig neu zu entwickelnde Neugeräte ersetzt werden, was ökonomisch nicht sinnvoll ist.

Eine Aufgabe der vorliegenden Erfindung kann daher darin gesehen werden, eine Abformmasse bereitzustellen, die einen hohen Widerstand beim Einsetzen des mit Abformmasse gefüllten Löffels in den Mund des Patienten gewährleistet und mit einem bekannten automatischen motorbetriebenen Misch- und Dosiersystem homogen gemischt werden kann, ohne die genannten Probleme zu zeigen.

Eine weitere Aufgabe kann darin gesehen werden, Abformmassen mit verminderter Viskosität bzw. deren Komponenten herzustellen, die nach dem Mischen einen putty-ähnlichen Charakter und nach dem Abbinden eine ausreichend hohe Shore-Härte aufweisen.

Demgemäß betrifft die vorliegende Erfindung eine zweikomponentige additionsvernetzende Silikonabformmasse, umfassend im gemischten Zustand der beiden Komponenten
(a) 1 bis 35 Gew.-% einer Mischung von mindestens zwei Organopolysiloxanen mit mindestens zwei ungesättigten Gruppen im Molekül, wobei
   mindestens ein Organopolysiloxan eine Viskosität im Bereich von 25 bis 1000 mPa*s und
   mindestens ein Organopolysiloxan eine Viskosität im Bereich von 60.000 bis 500.000 mPa*s,
   aufweist,
(b) 1 bis 10 Gew.-% mindestens eines Organohydrogenpolysiloxans mit mindestens zwei SiH-Gruppen im Molekül,
(c) 0,00005 bis 0,05 Gew.-% mindestens eines Platin-Katalysators, berechnet als elementares Platin,
(d) 4 bis 10 Gew.-% mindestens eines Paraffinöls oder mindestens eines weißen Mineralöls oder einer Mischung aus mindestens einem Paraffinöl und mindestens einem weißen Mineralöl,
(e) 50 bis 90 Gew.-% mindestens eines Füllstoffs,
jeweils bezogen auf das Gesamtgewicht der Silikonabformmasse, wobei eine der Komponenten vor dem Mischen mit der anderen Komponente eine Brookfield-Viskosität im Bereich von 800 bis 2000 Pa*s aufweist, dadurch gekennzeichnet, daß die Silikonabformmasse im gemischten Zustand eine nach ISO 4823 bestimmte Konsistenz von kleiner oder gleich 35 mm aufweist.

Die erfindungsgemäßen Silikonabformmassen sind für die maschinelle Mischung in üblichen motorbetriebenen automatischen Mischgeräten geeignet, ohne dass das Gerät selbst hierzu, wie oben beschrieben, abgewandelt werden muss. Im gemischten Zustand sind die Abformmassen verformbar und weisen eine nichtklebrige Konsistenz auf.

Das mindestens eine Organopolysiloxan gemäß (a) ist weiter bevorzugt in der erfindungsgemäßen Abformmasse in einem Anteil im Bereich von 5 bis 30 Gew.-% und besonders bevorzugt in einem Bereich von 10 bis 25 Gew.-% enthalten.

Das mindestens eine Organohydrogenpolysiloxan gemäß (b) ist weiter bevorzugt in der erfindungsgemäßen Abformmasse in einem Anteil im Bereich von 1 bis 9 Gew.-% und besonders bevorzugt in einem Bereich von 1 bis 8 Gew.-% enthalten.

Der mindestens eine Platin-Katalysator gemäß (c) ist weiter bevorzugt in der erfindungsgemäßen Abformmasse in einem Anteil im Bereich von 0,0001 bis 0,045 Gew.-% und besonders bevorzugt in einem Bereich von 0,0002 bis 0,05 Gew.-% enthalten.

Das mindestens eine Paraffinöl oder mindestens eine weiße Mineralöl oder die Mischung aus mindestens einem Paraffinöl und mindestens einem weißen Mineralöl gemäß (d) ist weiter bevorzugt in der erfindungsgemäßen Abformmasse in einem Anteil im Bereich von 4,5 bis 9 Gew.-% und besonders bevorzugt in einem Bereich von 5 bis 8 Gew.-% enthalten.

Der mindestens eine Füllstoff gemäß (e) ist weiter bevorzugt in der erfindungsgemäßen Abformmasse in einem Anteil im Bereich von 55 bis 85 Gew.-% und besonders bevorzugt in einem Bereich von 65 bis 83 Gew.-% enthalten.

Demgemäß betrifft die vorliegende Erfindung auch eine Silikonabformmasse, wie oben beschrieben, umfassend im angemischten Zustand der Komponenten
(a) 10 bis 25 Gew.-% einer Mischung von mindestens zwei Organopolysiloxanen mit mindestens zwei ungesättigten Gruppen im Molekül, wobei
   mindestens ein Organopolysiloxan eine Viskosität im Bereich von 25 bis 1000 mPa*s und
   mindestens ein Organopolysiloxan eine Viskosität im Bereich von 60.000 bis 500.000 mPa*s,
   aufweist,
(b) 1 bis 8 Gew.-% mindestens eines Organohydrogenpolysiloxans mit mindestens zwei SiH-Gruppen im Molekül,
(c) 0,0002 bis 0,04 Gew.-% Platin-Katalysator, berechnet als elementares Platin,
(d) 5 bis 8 Gew.-% mindestens eines Paraffinöls oder mindestens eines weißen Mineralöls oder eine Mischung aus mindestens einem Paraffinöl und mindestens einem weißen Mineralöl,
(e) 65 bis 83 Gew.-% mindestens eines Füllstoffs,
jeweils bezogen auf das Gesamtgewicht der Silikonabfomimasse.

Prinzipiell können die beiden Komponenten in allen beliebigen Volumenverhältnissen eingesetzt werden, solange die erfindungsgemäßen Abformmassen erhalten werden. Bevorzugt wird diejenige Komponente, die eine Brookfield-Viskosität im Bereich von 800 bis 2000 Pa*s aufweist, im Vergleich zur anderen Komponente in einem größeren Volumenanteil eingesetzt.

Demgemäß betrifft die vorliegende Erfindung auch eine Silikonabformmasse, wie oben beschrieben, die dadurch gekennzeichnet ist, daß diejenige Komponente, die vor dem Mischen mit der anderen Komponente eine Brookfield-Viskosität im Bereich von 800 bis 2000 Pa*s aufweist, mit einem größeren Volumenanteil als die andere Komponente eingesetzt wird.

Bevorzugt enthält die Komponente, die mit einem größeren Volumenanteil eingesetzt wird, keinen Katalysator.

In einer bevorzugten Ausführungsform werden die beiden Komponenten in einem Volumenverhältnis im Bereich von 4:1 bis 6:1, weiter bevorzugt im Bereich von 4:1 bis 5:1 und besonders bevorzugt von 4,4 :1 bis 4,6:1 eingesetzt.

Demgemäß betrifft die vorliegende Erfindung auch eine Silikonabformmasse, wie oben beschrieben, die dadurch gekennzeichnet ist, daß die beiden Komponenten in einem Volumenverhältnis im Bereich von 4:1 bis 6:1 eingesetzt werden.

In einer weiter bevorzugten Ausführungsform weist diejenige Komponente, die mit einem höheren Volumenanteil eingesetzt wird, eine Brookfield-Viskosität im Bereich von 900 bis 1800 Pa*s, besonders bevorzugt im Bereich von 950 bis 1700 und weiter besonders bevorzugt im Bereich von 1000 bis 1600 Pa*s auf.

Diejenige Komponente, die bevorzugt mit einem kleineren Volumenanteil eingesetzt wird, weist im allgemeinen eine Brookfield-Viskosität im Bereich von kleiner 1600 Pa*s, bevorzugt im Bereich von 300 bis 1400 Pa*s und besonders bevorzugt im Bereich von 500 bis 1200 Pa*s auf.

Überraschenderweise hat sich gezeigt, dass auch in den Fällen, in denen die beiden Komponenten unterschiedliche Viskosität aufweisen, deren homogene automatische Mischung möglich ist.

Prinzipiell können die Zusammensetzungen der beiden Komponenten beliebig gewählt werden, solange die erfindungsgemäßen Abformmassen mit den erfindungsgemäßen Eigenschaften erhalten werden.

Bevorzugt betrifft die vorliegende Erfindung dabei eine Silikonabformmasse, wie oben beschrieben, umfassend in der einen Komponente
(i) 15 bis 20 Gew.-% mindestens eines Organopolysiloxans mit mindestens zwei ungesättigten Gruppen im Molekül,
(ii) 1 bis 10 Gew.-% mindestens eines Organohydrogenpolysiloxans mit mindestens zwei SiH-Gruppen im Molekül,
(iii) 5 bis 8 Gew.-% mindestens eines Paraffinöls oder mindestens eines weißen Mineralöls oder einer Mischung aus mindestens einem Paraffinöl und mindestens einem weißen Mineralöl,
(iv) 60 bis 80 Gew.-% mindestens eines Füllstoffs,
jeweils bezogen auf das Gesamtgewicht dieser Komponente, wobei diese Komponente eine Brookfield-Viskosität im Bereich von 800 bis 2000 Pa*s aufweist, und in der anderen Komponente
(i') 5 bis 20 Gew.-% mindestens eines Organopolysiloxans mit mindestens zwei ungesättigten Gruppen im Molekül,
(ii') 0,00005 bis 0,05 Gew.-% mindestens eines Platin-Katalysators, berechnet als elementares Platin,
(iii') 0,5 bis 6 Gew.-% mindestens eines Paraffinöls oder mindestens eines weißen Mineralöls oder einer Mischung aus mindestens einem Paraffinöl und mindestens einem weißen Mineralöl,
(iv') 60 bis 80 Gew.-% mindestens eines Füllstoffs,
jeweils bezogen auf das Gesamtgewicht dieser Komponente, wobei entweder der Bestandteil (a) oder der Bestandteil (i) oder der Bestandteil (i') oder sowohl der Bestandteil (i) als auch der Bestandteil (i') mindestens zwei voneinander verschiedene Organopolysiloxane mit mindestens zwei ungesättigten Gruppen im Molekül umfaßt, wobei
- mindestens ein Organopolysiloxan eine Viskosität im Bereich von 25 bis 1000 mPa*s und
- mindestens ein Organopolysiloxan eine Viskosität im Bereich von 60.000 bis 500.000 mPa*s
aufweist.

Was den Bestandteil gemäß (a) oder (i) und (i') anbelangt, so kann dieser aus mehreren, voneinander verschiedenen Organopolysiloxanen bestehen. So kann beispielsweise der Bestandteil gemäß (i) ein Organopolysiloxan und der Bestandteil gemäß (i') das gleiche oder ein davon verschiedenes Organopolysiloxan enthalten. Ebenso ist es möglich, daß beispielsweise der Bestandteil gemäß (i) oder (i') ein Organopolysiloxan und der Bestandteil gemäß (i') oder (i) zwei oder mehr Organopolysiloxane, von denen beispielsweise eines gleich dem des Bestandteils (i) oder (i') sein kann, enthalten. Ebenso ist es möglich, daß der Bestandteil gemäß (i) zwei oder mehr Organopolysiloxane und der Bestandteil gemäß (i') zwei oder mehr Polysiloxane enthält, wobei sämtliche Organopolysiloxane voneinander verschieden sein können.

Besonders bevorzugt ist weiterhin die Verwendung einer Kombination von mindestens zwei linearen dimethylvinylsiloxyterminierten Polydimethylsiloxanen einer Struktur, wie sie untenstehend beschrieben ist, mit verschiedener Viskosität.

Die Kombination umfasst bevorzugt einen Vertreter hoher Viskosität, bevorzugt im Bereich von 60.000 mPa*s bis 500.000 mPa*s, besonders bevorzugt im Bereich von 70.000 bis 200.000 mPa*s, und weiter besonders bevorzugt im Bereich von 80.000 bis 150.000 mPa*s und einen Vertreter niedriger Viskosität, bevorzugt im Bereich von 25 bis 1.000 mPa*s, besonders bevorzugt im Bereich von 50 bis 500 mPa*s und weiter besonders bevorzugt im Bereich von 100 bis 300 mPa*s.

In einer weiter bevorzugten Ausführungsform betrifft die vorliegende Erfindung eine Silikonabformmasse, wie oben beschrieben, die dadurch gekennzeichnet ist, daß Bestandteil (a) das mindestens eine Organopolysiloxan mit einer Viskosität im Bereich von 25 bis 1000 mPa*s in einem Anteil im Bereich von 1 bis 5 Gew.-% und das mindestens eine Organopolysiloxan mit einer Viskosität im Bereich von 60.000 bis 500.000 mPa*s in einem Anteil im Bereich von 10 bis 20 Gew.-% enthält.

In einer ebenfalls bevorzugten Ausführungsform enthält der Bestandteil gemäß (a) auch mindestens ein Organopolysiloxan mit einer mittleren Viskosität im Bereich von 2000 bis 50.000 mPa*s, bevorzugt im Bereich von 5.000 und 20.000 mPa*s, und besonders bevorzugt im Bereich von etwa 10.000 mPa*s. Dabei ist es unter anderem möglich, daß dieses mindestens eine Organopolysiloxan entweder im Bestandteil gemäß (i) oder im Bestandteil gemäß (i') oder in diesen beiden Bestandteilen enthalten ist, wobei auch zwei oder mehr voneinander verschiedene dieser Organopolysiloxane eingesetzt werden können.

Die Verhältnisse der Gewichtsanteile des niedrigviskosen Vertreters gemäß Bestandteil (a) zum hochviskosen Vertreter gemäß Bestandteil (a) liegen im allgemeinen im Bereich von 1:2 bis 1:20, bevorzugt im Bereich von 1:2,5 bis 1:10, besonders bevorzugt im Bereich von 1:3 bis 1:8 und ganz besonders bevorzugt im Bereich von 1:3 bis 1:5. Falls zusätzlich ein mittelviskoser Vertreter gemäß (a) eingesetzt wird, liegen die Gewichtsverhältnisse dieses Vertreters zum hochviskosen Vertreter bevorzugt im Bereich von 1:2,5 bis 1:10, besonders bevorzugt im Bereich von 1:3 bis 1:8 und ganz besonders bevorzugt im Bereich von 1:5 bis 1:8.

Bei dem Bestandteil gemäß (a) beziehungsweise (i) und (i') handelt es sich bevorzugt um mindestens ein Diorganopolysiloxan mit terminalen Triorganosiloxygruppen, von denen mindestens eine eine Vinylgruppe ist.

Bevorzugte Diorganopolysiloxane dieser Struktur werden durch die folgende Formel wiedergegeben,

CH₂=CH(-SiR₂-O)ₙ-SiR₂-CH=CH₂

in der R eine unsubstituierte oder substituierte monovalente Kohlenwasserstoffgruppe repräsentiert, die bevorzugt frei von aliphatischen Mehrfachbindungen ist und n eine ganze Zahl darstellt. Bevorzugt sind mindestens 50% der Reste R Methylgruppen. Beispiele für andere Gruppen R sind Ethyl-, Vinyl-, und 3,3,3-Trifluorpropylgruppen. Der Wert von n wird bevorzugt so gewählt, dass das Polymer bei 25°C eine Viskosität im Bereich von 25 bis 500.000 mPa*s aufweist. Derartige Moleküle sind in der US 4,035,453 beschrieben, deren Offenbarung diesbezüglich in die vorliegende Anmeldung vollumfänglich einbezogen wird.

Die Herstellung der Bestandteile gemäß (a) oder (i) oder (i') erfolgt nach üblichen Verfahren, die beispielsweise in W. Noll, "Chemie und Technologie der Silikone", Verlag Chemie Weinheim, 2. Auflage 1964, Seite 162 - 206, dargestellt sind.

Besonders bevorzugt sind lineare Polydimethylsiloxane obiger Struktur mit den angegebenen Viskositätsbereichen, bei denen die Endgruppen aus Dimethylvinylsiloxyeinheiten bestehen und die weiteren Substituenten R in der Kette aus Methylgruppen bestehen.

Der Vertreter mit einer niedrigen Viskosität liegt vorzugsweise in einem Anteil im Bereich von 1 bis 5 Gew.-%, bezogen auf das Gesamtgewicht der gemischten Silikonabformmasse, vor.

Die einsetzbaren Füllstoffe (e) sind bevorzugt nicht-verstärkende Füllstoffe mit einer BET-Oberfläche von bis zu 50 m²/g, wie Quarz, Cristoballit, Calciumsilikat, Zirkoniumsilikat, Montmorillonite, Benthonite, Zeolithe, einschließlich der Molekularsiebe, wie Natriumaluminiumsilikat, Metalloxidpulver, wie Aluminium- oder Zinkoxide bzw. deren Mischoxide, Bariumsulfat, Calciumcarbonat, Gips, Glas- und Kunststoffpulver. Besonders bevorzugt sind Quarz, Cristoballit und Natriumaluminiumsilikate, die oberflächenbehandelt sein können.

Demgemäß betrifft die vorliegende Erfindung auch eine Silikonabformmasse, wie oben beschrieben, die dadurch gekennzeichnet ist, daß der mindestens eine Füllstoff gemäß (e) eine BET-Oberfläche von bis zu 50 m²/g aufweist.

In einer besonders bevorzugten Ausführungsform weisen die Füllstoffe gemäß (e) eine BET-Oberfläche von bis zu 10 m²/g auf.

Sämtliche der in dieser Anmeldung angegebenen BET-Oberflächen beziehen sich auf Messungen, die gemäß DIN 66132 durchgeführt wurden.

Zu möglichen Füllstoffen gehören auch verstärkende Füllstoffe mit einer BET-Oberfläche von größer als 50 m²/g, wie pyrogene oder gefällte Kieselsäure wie beispielsweise Aerosil, und Siliciumaluminium-Mischoxide.

Im Rahmen der vorliegenden Erfindung ist es beispielsweise möglich, sowohl verstärkende als auch nicht-verstärkende Füllstoffe einzusetzen, wobei beispielsweise in der einen Komponente mindestens ein verstärkender und in der anderen Komponente mindestens ein nicht-verstärkender Füllstoff vorliegt. Ebenso können auch verstärkender und nicht-verstärkender Füllstoff in einer einzigen Komponente zusammen oder in beiden Komponenten vorliegen, wobei in den unterschiedlichen Komponenten voneinander verschiedene verstärkende beziehungsweise verschiedene nicht-verstärkende Füllstoffe enthalten sein können.

In einer bevorzugten Ausführungsform der erfindungsgemäßen Abformmassen werden die Füllstoffe derart ausgewählt, daß der mindestens eine verstärkende Füllstoff in einem Anteil im Bereich von 0,00001 bis 5 %, bezogen auf den Gesamtfüllstoffgehalt der Abformmasse, vorliegt. Weiter bevorzugt liegt dieser Anteil im Bereich von 0,1 bis 4 % und besonders bevorzugt in einem Bereich von 0,3 bis 3 %.

Die genannten Füllstoffe können oberflächenbehandelt und dabei bevorzugt hydrophobiert sein, beispielsweise durch die Behandlung mit Organosilanen oder - siloxanen oder durch die Veretherung von Hydroxylgruppen zu Alkoxylgruppen.

Die Füllstoffmengen sind grundsätzlich so auszuwählen, dass eine Shorehärte A der ausgehärteten Silikonabformmassen erreicht wird, die im Bereich von 40 bis 80, bevorzugt im Bereich von 60 bis 75 liegt.

Sämtliche der in dieser Anmeldung angegebenen Werte der Shore-Härte A verstehen sich als Werte, die gemäß DIN 53505 bestimmt werden.

Auch die oben genannten Füllstoffe können zur Einstellung der rheologischen Eigenschaften der Silikonabformmassen verwendet werden. Weiter ist es möglich, auch die einzelnen Komponenten, die Basis- und die Katalysatorkomponente, in ihren jeweiligen rheologischen Eigenschaften zu steuern.

Ein Vorteil der Verwendung von mindestens zwei verschieden viskosen Bestandteilen gemäß (a) beziehungsweise (i) und (i'), wie sie oben beschrieben sind, ist unter anderem darin zu sehen, daß die Einstellung der rheologischen Eigenschaften der Silikonabformmassen ermöglicht wird. Weiter ist es möglich, auch die einzelnen Komponenten, die Basis- und die Katalysatorkomponente, in ihren jeweiligen rheologischen Eigenschaften zu steuern.

Demgemäß betrifft die vorliegende Erfindung auch die Verwendung
- einer Mischung von mindestens zwei Organopolysiloxanen mit mindestens zwei ungesättigten Gruppen im Molekül, wobei
   -- mindestens ein Organopolysiloxan eine Viskosität im Bereich von 25 bis 1000 mPa*s und
   -- mindestens ein Organopolysiloxan eine Viskosität im Bereich von 60.000 bis 500.000 mPa*s,
   aufweist, oder
- von sowohl dieser mindestens zwei Organopolysiloxane als auch mindestens eines Füllstoffs mit einer BET-Oberfläche von bis zu 50 m²/g,
als Bestandteile einer Silikonabformmasse oder mindestens einer der beiden Komponenten, wie oben beschrieben, zur Einstellung der rheologischen Eigenschaften der Silikonabformmasse oder Komponenten.

Insbesondere beschreibt die vorliegende Erfindung die Verwendung dieser Organopolysiloxanen zur Einstellung der rheologischen Eigenschaften von zweikomponentigen additionsvernetzenden Silikonabformmassen vom Typ nach ISO 4823, wodurch erreicht werden kann, dass diese sich mit motorgetriebenen Mischsystemen ausbringen lassen, was insbesondere bei vorherrschenden Ausdrückkräften von bis zu 4000 N der Fall ist. Es hat sich gezeigt, dass zur Erzielung eines brauchbaren Mischergebnisses ohne mechanische Beeinträchtigung des Mischgeräts, der Kartuschen, der Schlauchbeutel oder des Mischers die Förderkraft, die über die Kolben auf die zu mischenden Pasten übertragen wird, vorteilhafterweise den Betrag von etwa 4000 N nicht wesentlich übersteigen sollte.

Der Betrag der Ausdrückkraft wird in einer Universalprüfmaschine ZWICK bestimmt. Mit Basis- und Katalysatorpaste befüllte Schlauchbeutel werden in die entsprechenden Kartuschen eingelegt. Die befüllte Kartusche wird in eine Prüfvorrichtung eingelegt, die im wesentlichen aus einem elektrisch betriebenen Ausbringgerät besteht, das in eine Universalprüfmaschine (Zwick) eingespannt ist.

Die Messung wird folgendermaßen durchgeführt: Die Kraft zum Befördern der Paste, die auf die Kolbenscheiben des Ausbringgeräts wirkt, wird nicht über den Motor des ursprünglichen Ausbringgeräts ausgeübt, sondern über die Vorschubeinheit der Universalprüfmaschine. Dabei kann die ausgeübte Gesamtkraft gemessen werden (Drucksensor, 10 KN-Messdose, Vorschubgeschwindigkeit 23 mm/min). Bei der Messung wird ein Mischer gemäß der deutschen Patentanmeldung DE-A 101 12 904.1 verwendet. Dieser wird über die Mischerwelle des Ausbringgeräts angetrieben. Für die Ermittlung der Ausdrückkraft ist derjenige Wert relevant, der sich nach einer Vorschubstrecke von 30 - 50 mm ergibt.

Daher betrifft die vorliegende Erfindung auch die beschriebene Verwendung, die dadurch gekennzeichnet ist, daß die rheologischen Eigenschaften zu Ausdrückkräften in einem zum Ausbringen der Komponenten und der Silikonabformmasse verwendeten automatischen Mischgerät von höchstens 4000 N führen

Überraschenderweise wurde gefunden, dass sich durch Zusetzen einer Mischung von Organopolysiloxanen, die mindestens eine reaktive Vinylgruppe und bestimmte Viskositätswerte aufweisen, als Bestandteil zu einer zweikomponentigen additionsvernetzenden Silikonabformmasse, sich die Gesamtviskosität der gemischten Paste derart steuern und insbesondere herabsetzen lässt, dass sich die Komponenten bzw. Massen in einem elektrisch betriebenen Mischgerät automatisch mischen und ausbringen lassen, ohne dass sie den Putty-Charakter (Formbarkeit, NichtKlebrigkeit, Einbringwiderstand) zu verlieren.

Der Widerstand beim Einsetzen des mit gemischter Abformmasse befüllten Abformlöffels kann näherungsweise beschrieben werden durch den Widerstand, der durch die höhervolumige Paste alleine ausgeübt wird. Diese Betrachtung bietet den Vorteil, dass die Beiträge der niedervolumigen Paste eliminiert werden, die volumenmäßig bei einem 4,5:1-System nur einen relativ geringen Anteil an der gemischten Paste hat.

Der Widerstand der höhervolumigen Paste beim Einsetzen des Löffels kann durch die Viskositätsmessung nach Brookfield modellhaft beschrieben werden, bei der der Widerstand der Paste gegen die Rotationsbewegung eines eingebrachten Metallkreuzes mit definierten Balkenlängen gemessen wird. Die Viskosität lässt sich beispielsweise mit einem Brookfield DV III Rheometer (Spindel HB 5, Balkenbreite 15,5 mm, Geschwindigkeit 5 U/min, Ablesung 20 sec nach Start) bestimmen.

Konkret wird die Viskosität bei 23 °C auf folgende Weise bestimmt werden: Das Rheometer wird nach dem Einschalten und der Justierung mit der Mess-Spindel HB5 (Balkenbreite = 15,5 mm) versehen. Danach wird die zu messende Paste mit dem größeren Volumenanteil zunächst 40 sec lang mit den Händen geknetet und dann blasenfrei in einen Messbecher gefüllt; in Schlauchbeuteln verpacktes Material wird direkt aus einem handelsüblichen Mischgerät (Pentamix® 2, Firma ESPE) in einen Messbecher gefüllt, dessen Durchmesser mindestens 20 mm beträgt, zur Vermeidung von Effekten, die von der Becherwandung herrühren können. Die Füllhöhe soll mindestens 30 mm betragen. Ohne Zeitverzug wird danach der befüllte Messbecher in das Becheraufnahmestativ gestellt, und die Messspindel mittig 25 mm in die Paste abgesenkt. Nun wird die Prüfung gestartet. Die Ablesung des im Display als Centipoise (entspricht mPa*s) angezeigten Messwertes erfolgt 20 sec nach Start der Messung. Zu beachten ist, dass zu keiner Zeit der Messung der Messbecher durch die Rotation der Spindel mitgezogen werden darf. Dabei wird ein Viskositätswert in Pa*s erhalten, der ein direktes Maß für den Widerstand darstellt, den die Paste einer Bewegung gegen einen festen Körper entgegensetzt. Man verwendet einen Mittelwert aus mindestens 5 Messungen. Dieser Wert korreliert mit dem Widerstand gegen das Einsetzen des Abformlöffels, bei dem die festen Körper die Zähne des Patienten darstellen. Es ist aber auch eine Korrelation gegeben mit dem Widerstand, den die Paste gegen das Fördern und Mischen in automatischen motorgetriebenen Mischgeräten entgegensetzt, da auch hier eine Bewegung und mehrfache Umlenkung der Strömungsrichtung der Paste durch die Strömungskanäle und die Mischflügel innerhalb des Mischgeräts erfolgt.

Unter dem Begriff "Brookfield-Viskosität" wird im Rahmen der vorliegenden Anmeldung eine Viskosität verstanden, die wie vorstehend beschrieben gemessen wurde.

Die Viskositätsangaben der Bestandteile (a), (b) und (d) verstehen sich als dynamische Viskositäten, die gemäß DIN 53018 bzw. 51562 bei 20 °C gemessen werden, wobei die Viskositäten der Bestandteile gemäß (a) und (b) gemäß DIN 53018 und die Bestandteile gemäß (d) gemäß DIN 51562 bestimmt werden.

Der Bestandteil gemäß (b) ist bevorzugt mindestens ein Organopolysiloxan mit mindestens 3 Si-gebundenen Wasserstoffatomen pro Molekül. Dieses Organopolysiloxan enthält bevorzugt 0,01 bis 2 Gew.-% siliciumgebundenen Wasserstoff. Die Silikonvalenzen, die nicht mit Wasserstoff- oder Sauerstoffatomen abgesättigt sind, werden mit monovalenten Kohlenwasserstoffradikalen abgesättigt, die frei von aliphatischen Mehrfachbindungen sind. Die Kohlenwasserstoffradikale können substituiert oder unsubstituiert sein. Mindestens 50%, bevorzugt 100% der Kohlenwasserstoffradikale, die an Siliciumatomen gebunden sind, bestehen aus Methylradikalen. Auch derartige Komponenten sind in den oben genannten Literaturstellen hinsichtlich Struktur und Herstellung beschrieben.

Die Mengenverhältnisse der Bestandteile (a) und (b) sind bevorzugt derart gewählt, dass pro Mol an ungesättigter Doppelbindung gemäß (a) 0,75 bis 5 Mol SiH-Einheiten gemäß (b) vorliegen. Die Summe der Bestandteile (a) und (b) liegen im Bereich von 10 bis 40 Gew.-%, bevorzugt von 15 bis 25 Gew.-%, bezogen auf das Gesamtgewicht aller Bestandteile der Silikonabformmasse.

Der Bestandteil gemäß (c) ist bevorzugt ein Platinkomplex, der, wiederum bevorzugt, aus Hexachloroplatinsäure durch Reduktion mit Tetramethyldivinyldisiloxan hergestellt werden kann. Geeignet sind auch andere Platinverbindungen, die die Additionsvernetzung beschleunigen. Gut geeignet sind beispielsweise Platin-Siloxan-Komplexe, wie sie in US 3,715,334, US 3,775,352 und US 3,814,730 beschrieben sind. Der Platinkatalysator wird vorzugsweise in Mengen im Bereich von 0,00005 bis 0,05 Gew.-%, insbesondere im Bereich von 0,0002 bis 0,04 Gew.-%, besonders bevorzugt im Bereich von 20 bis 100 Gewichts-ppm und weiter besonders bevorzugt im Bereich von 25 bis 60 Gewichts-ppm, jeweils berechnet als elementares Platin, bezogen auf das Gesamtgewicht aller Bestandteile der Silikonabformmasse, eingesetzt. Sollte es zweckmäßig sein, können zwei oder mehr Platinkatalysatoren eingesetzt werden.

Zur Steuerung der Reaktivität kann ein Inhibitor zugesetzt werden, der die vorzeitige Vernetzung zum Elastomeren behindert. Derartige Inhibitoren sind beispielsweise in der US 3,933,880 beschrieben. Beispiele hierfür sind unter anderem acetylenisch ungesättigte Alkohole, wie 3-Methyl-1-butin-3-ol, 1-Ethyinylcyclohexan-1-ol, und 3-Methyl-1-pentin-3-ol. Beispiele für Inhibitoren auf Vinylsiloxanbasis sind unter anderem 1,1,3,3-Tetramethyl-1,3-divinyldisiloxan und vinylgruppenhaltige Oligo- und Disiloxane, die bevorzugt in Mengen im Bereich von 0,00001 bis 0,2 Gew.-%, bezogen auf des Gesamtgewicht aller Bestandteile der Silikonabformmasse, zugesetzt werden.

Weiterhin können die erfindungsgemäßen Abformmassen gegebenenfalls mindestens einen üblichen Zusatzstoff enthalten. Solche üblichen Zusatzstoffe sind beispielsweise Weichmacher, Silikonöle, Pigmente, Antioxidationsmittel oder Trennmittel. Ebenso können auch fein verteiltes Palladium oder Platin als Wasserstoffabsorber enthalten sein. Die Metalle können dabei, falls erforderlich, auch auf geeignete Trägermaterialien aufgebracht sein. Solch übliche Zusatzstoffe sind in der erfindungsgemäßen Abformmasse bevorzugt in einem Anteil im Bereich von 0,0001 bis 2 Gew.-% enthalten.

Demgemäß betrifft die vorliegende Erfindung auch eine Silikonabformmasse, wie oben beschrieben, die dadurch gekennzeichnet ist, daß sie zusätzlich Inhibitor in einem Anteil im Bereich von 0,00001 bis 0,2 Gew.-% oder mindestens einen üblichen Zusatzstoff in einem Anteil im Bereich von 0,00001 bis 2 Gew.-% oder eine Mischung aus Inhibitor und mindestens einem üblichen Zusatzstoffe in einem Anteil von 0,00002 bis 2,2 Gew.-%, jeweils bezogen auf das Gesamtgewicht der Silikonabformmasse, umfaßt.

Der Bestandteil gemäß (d) ist mindestens ein Paraffinöl oder weißes Mineralöl oder ein Gemisch davon, das ein bei Raumtemperatur flüssiges Gemisch von Alkanen mit einer Viskosität von vorzugsweise 100 bis 400 mPa*s bei 20°C, besonders bevorzugt 110 bis 300 mPa*s und weiter besonders bevorzugt zwischen 120 bis 250 mPa*s darstellt. Bevorzugt werden dabei Qualitäten eingesetzt, die beispielsweise durch Hydrierung von aromatischen polycyclischen Kohlenwasserstoffen weitgehend befreit sind und die vorwiegend eine Kohlenstoff-Kettenverteilung zwischen ca. 15 und 50 Kohlenstoffatomen aufweisen.

Demgemäß betrifft die vorliegende Erfindung auch eine Silikonabformmasse, wie oben beschrieben, die dadurch gekennzeichnet ist, daß das mindestens eine Paraffinöl oder das mindestens eine weiße Mineralöl oder die Mischung aus mindestens einem Paraffinöl und mindestens einem weißen Mineralöl gemäß (d) bei 20 °C eine Viskosität im Bereich von 100 bis 400 mPa*s aufweist.

Eine Nichtklebrigkeit der gemischten Paste ist überraschenderweise möglich, obwohl beispielsweise die niedervolumige Paste alleine an den Händen kleben kann.

Erfindungsgemäß ist ebenfalls ein Verfahren zum Mischen von gemäß ISO 4823 knetbaren Abformmassen mittels automatischen motorbetriebenen Mischgeräten.

Daher betrifft die vorliegende Erfindung auch ein Verfahren zum Anmischen einer Silikonabformmasse, das dadurch gekennzeichnet ist, daß das Mischen der beiden Komponenten unter Verwendung eines automatischen motorbetriebenen Mischgerätes erfolgt.

Insbesondere betrifft die vorliegende Erfindung dabei ein Verfahren, wie oben beschrieben, das dadurch gekennzeichnet ist, daß es folgende Schritte umfaßt:
(A) Einbringen der in Primärverpackungen enthaltenen Komponenten in Sekundärverpackungen, die mit den Abmessungen des automatischen Mischgerätes kompatibel sind, oder
   Einbringen der in Primärverpackungen enthaltenen Komponenten direkt in das automatische Mischgerät, oder
   Einbringen der in einer Primärverpackung enthaltenen einen Komponente in eine Sekundärverpackung, die mit den entsprechenden Abmessungen des automatischen Mischgerätes kompatibel ist, und direktes Einbringen der in einer Primärverpackung enthaltenen Komponente in das automatische Mischgerät;
(B) Ausbringen der Komponenten aus dem automatischen Mischgerät über eine dynamische Mischkanüle unter Vermischung der Komponenten bei Ausdrückkräften von höchstens 4000 N.

### Herstellungsbeispiel

In einem üblichen Laborkneter wurde eine Basispaste hergestellt, indem bis zur Homogenität 17,1 Gewichtsteile eines vinylterminierten Polydimethylsiloxans mit einer Viskosität von 100.000 mPa*s, 1,8 Teile eines vinylterminierten Polydimethylsiloxans mit einer Viskosität von 200 mPa*s, 3,6 Teile eines Polymethylhydrogensiloxans mit einer Viskosität von 50 mPa*s und 6,6 Teile eines Paraffinöls einer Viskosität von 130 mPa*s mit 70,7 Gewichtsteilen eines hydrophobierten Quarz-Pulvers mit einer mittleren Korngröße von 4 µm und 0,2 Teilen der Farbpaste FL Dunkelblau (Wacker Chemie) vereinigt wurden.

Eine Katalysatorpaste wurde gefertigt durch Mischen von 13,3 Teilen eines vinylterminierten Polydimethylsiloxans mit einer Viskosität von 10.000 mPa*s, 5,6 Teilen eines vinylterminierten Polydimethylsiloxans mit einer Viskosität von 200 mPa*s, 4 Teilen eines Paraffinöls mit einer Viskosität von 130 mPa*s, 2,2 Teilen einer Lösung einer Platinkomplexmischung in Silikonöl mit einem Gewichtsgehalt von etwa 1 % an Platin, 0,9 Teilen eines anorganischen Farbpigments (Sicomet Grün, BASF) mit 74 Teilen eines Natrium-Silikat-Füllstoffes einer mittleren Korngröße von 2 µm.

### Anwendungsbeispiel

300 ml Basis- und 66 ml Katalysatorpaste gemäß Herstellungsbeispiel wurden in Schlauchbeutel abgefüllt. Anschließend wurden diese Schlauchbeutel in ein Pentamix® 2 Mischgerät der Fa. ESPE, Seefeld, eingebracht. Bei der Verwendung eines Mischers nach der Patentanmeldung DE-A 101 12 904.1 konnten beide Pasten in einer homogenen Mischung aus dem Gerät gefördert werden, ohne dass es zum Durchrutschen der Gerätekupplung kam. Die Brookfield-Viskosität der Basispaste betrug 1.500 Pa*s. Vor dem Mischen war die Basispaste an den Fingern nicht klebrig, die Katalysatorpaste hingegen haftete an den Fingern. Die gemischte Paste war nicht klebrig und konnte im Abformlöffel mit den Fingern modelliert werden, obwohl die Katalysatorpaste alleine an den Fingern haftete. Im gemischten Zustand war nach etwa 2 min eine spürbare Viskositätserhöhung feststellbar. Nach etwa 6 min lag ein ausgehärteter Gummi vor. Die Shore-Härte A des Gummis (gemessen nach DIN 53 505) betrug 70, gemessen 30 min nach dem Mischen. Die nach ISO 4823 bestimmte Konsistenz betrug 28 mm.

### Vergleichsversuche

Vergleichsversuche mit im Markt erhältlichen Knetmassen zeigten (Tabelle 1), dass deren höhervolumige Pasten Brookfield-Viskositäten von mindestens 2.000 Pa*s aufweisen. Der vorher beschriebene Versuch zum Ausbringen der Pasten aus einem automatischen Mischgerät führte zum Versagen des Gerätes.

**Tabelle 1**

| **Produkt** (Hersteller) | **Brookfield-Viskosität** | | **Konsistenz nach ISO 4823 [mm]** | **Chargen-Nr.** |
|---|---|---|---|---|
| | **Basis- Paste** | **Kat.- Paste** | | |
| Dimension® Penta H Quick (3M ESPE AG) * # | 744 | 600 | 29 | 254/2002.01 |
| Silagum® Putty Soft (DMG) * # | 770 | | 31 | 90023 / 2002.03 |
| Herstellungsbeispiel # | 1480 | 640 | 28 | 002 |
| Blend-A-Gum® Body (Densply) | 2160 | 3624 | 25 | 6000177/2001.10 |
| Reprosil® Easy Mix Putty (Dentsply) | 2370 | 3000 | 27 | 3001948 / 2002.09 |
| Permagum® (3M ESPE AG) | 2640 | | 26 | 081/2002.12 |
| Flexitime® Easy Putty (Heraeus Kulzer) | 3008 | 2770 | 29 | 130342 / 2002.04 |
| Exaflex® Putty | 3320 | 2680 | 26 | 121198A |
| (GC) | | | | |
| Panasil Putty (Kettenbach) | 3790 | 2632 | 25 | 6898 |

- Alle Brookfield-Viskositätsangaben in Pa*s.
- Bei den mit * markierten Vergleichsprodukten handelt es sich um handelsübliche Silikonabformmassen, die aus dem Pentamix® 2 - Mischgerät gefördert wurden, aber keinen hohen Einbringwiderstand aufweisen und im gemischten Zustand an den Fingern kleben, also nicht mit den Fingern formbar waren.
- Bei mit # gekennzeichneten Produkten handelt es sich um Produkte, die in dem Volumenverhältnis 4,5:1 im Pentamix®-Mischgerät gemischt wurden.
- Die nicht mit # gekennzeichneten Produkte sind handelsübliche Putty-Knetmassen im Volumenverhältnis von 1:1.

Beispielhaft wird ein Ausdrückversuch mit dem Produkt Blend-A-Gum (Ch. # 0006000177) geschildert.

Basis- und Katalysatorpaste wurden in Schlauchbeutel abgefüllt, die mit dem ESPE Pentamix®-System kompatibel sind, wobei das Volumenverhältnis von Basis zu Katalysator 4,5:1 betrug. Die Schlauchbeutel wurden verschlossen und in handelsübliche Pentamix®-Kartuschen eingelegt, nachdem sie mit standardisierten, im Pentamix®-Gerät selbstöffnenden Auslasskappen gemäß DE-A 296 06 895 versehen worden waren (Öffnungsdurchmesser Einlass für Basispaste 8,6 mm, Einlass für Katalysatorpaste 2,3 mm). Die Pasten wurden mit Hilfe eines Pentamix® 2-Gerätes unter Verwendung eines Mischers nach DE-A 101 12 904.1 ausgedrückt. Der Mischer entspricht dem Mischer, der in der EP-A 0 993 863 beschrieben ist.

Zum Vergleich wurde die erfindungsgemäße Paste nach obigem Herstellungsbeispiel unter Verwendung des gleichen Systems ausgedrückt.

Nachdem 20 g des Vergleichsmaterials aus dem Mischgerät ausgebracht waren, war bereits ein deutlicher Temperaturanstieg im Gerät auf ca. 40 °C zu verzeichnen.

Die vollständige Füllung eines Oberkiefer-Abformlöffels (ca. 40 g Materialmenge) war nicht möglich, da das anzumischende Material bereits in der Mischkanüle abzubinden begann und die Förderung der Pasten durch die Mischmaschine aufgrund zu hoher Kräfte nicht mehr funktionierte. Die Ausdrückkräfte betrugen nach einer Fördermenge von 20 g mehr als 4900 N.

Das erfindungsgemäße Material gemäß Anwendungsbeispiel hingegen ließ sich problemlos ausdrücken und Mischen, wobei eine Temperaturspitze von 35 °C beobachtet und eine Förderrate von 130 g/min erzielt wurde. Die Mischqualität war homogen und blasenfrei. Die aufzubringenden Ausdrückkräfte betrugen bei Verwendung des oben genannten Mischers 3500 N.

## Patentansprüche

1. Zweikomponentige additionsvernetzende Silikonabformmasse, umfassend im gemischten Zustand der beiden Komponenten
(a) 1 bis 35 Gew.-% einer Mischung von mindestens zwei Organopolysiloxanen mit mindestens zwei ungesättigten Gruppen im Molekül, wobei
mindestens ein Organopolysiloxan eine Viskosität im Bereich von 25 bis 1000 mPa*s und
mindestens ein Organopolysiloxan eine Viskosität im Bereich von 60.000 bis 500.000 mPa*s,
aufweist.
(b) 1 bis 10 Gew.-% mindestens eines Organohydrogenpolysiloxans mit mindestens zwei SiH-Gruppen im Molekül,
(c) 0,00005 bis 0,05 Gew.-% mindestens eines Platin-Katalysators, berechnet als elementares Platin,
(d) 4 bis 10 Gew.-% mindestens eines Paraffinöls oder mindestens eines weißen Mineralöls oder einer Mischung aus mindestens einem Paraffinöl und mindestens einem weißen Mineralöl,
(e) 50 bis 90 Gew.-% mindestens eines Füllstoffs,
jeweils bezogen auf das Gesamtgewicht der Silikonabformmasse, wobei eine der Komponenten vor dem Mischen mit der anderen Komponente eine Brookfield-Viskostät im Bereich von 800 bis 2000 Pa*s aufweist, **dadurch gekennzeichnet, daß** die Silikonabformmasse im gemischten Zustand eine nach ISO 4823 bestimmte Konsistenz von kleiner oder gleich 35 mm aufweist.

2. Silikonabformmasse gemäß Anspruch 1, umfassend im gemischten Zustand der Komponenten
(a) 10 bis 25 Gew.-% einer Mischung von mindestens zwei Organopolysiloxanen mit mindestens zwei ungesättigten Gruppen im Molekül, wobei
mindestens ein Organopolysiloxan eine Viskosität im Bereich von 25 bis 1000 mPa*s und
mindestens ein Organopolysiloxan eine Viskosität im Bereich von 60.000 bis 500.000 mPa*s,
aufweist.
(b) 1 bis 8 Gew.-% mindestens eines Organohydrogenpolysiloxans mit mindestens zwei SiH-Gruppen im Molekül,
(c) 0,0002 bis 0,04 Gew.-% Platin-Katalysator, berechnet als elementares Platin.
(d) 5 bis 8 Gew.-% mindestens eines Paraffinöls oder mindestens eines weißen Mineralöls oder einer Mischung aus mindestens einem Paraffinöl und mindestens einem weißen Mineralöl,
(e) 65 bis 83 Gew.-% mindestens eines Füllstoffs,
jeweils bezogen auf das Gesamtgewicht der Silikonabformmasse.

3. Silikonabformmasse gemäß.Anspruch 1 oder 2, **dadurch gekennzeichnet, daß** diejenige Komponente, die vor dem Mischen mit der anderen Komponente eine Brookfield-Viskosität im Bereich von 800 bis 2000 Pa*s aufweist, mit einem größeren Volumenanteil als die andere Komponente eingesetzt wird.

4. Silikonabformmasse gemäß einem der Ansprüche 1 bis 3, **dadurch gekennzeichnet, daß** die beiden Komponenten in einem Volumenverhältnis im Bereich von 4:1 bis 6:1 eingesetzt werden.

5. Silikonabformmasse gemäß einem der Ansprüche 1 bis 4, zusätzlich umfassend mindestens einen Inhibitor mit einem Anteil im Bereich von 0,00001 bis 0,2 Gew.-% oder mindestens einen üblichen Zusatzstoff mit einem Anteil im Bereich von 0,00001 bis 2 Gew.-% oder eine Mischung aus mindestens einem Inhibitor und mindestens einem üblichen Zusatzstoff mit einem Anteil von 0,00002 bis 2,2 Gew.-%, jeweils bezogen auf das Gesamtgewicht der Silikonabformmasse.

6. Silikonabformmasse gemäß einem der Ansprüche 1 bis 5, **dadurch gekennzeichnet, daß** das mindestens eine Paraffinöl oder das mindestens eine weiße Mineralöl oder die Mischung aus mindestens einem Paraffinöl und mindestens einem weißen Mineralöl gemäß (d) bei 20 °C eine Viskosität im Bereich von 100 bis 400 mPa*s aufweist.

7. Silikonabformmasse gemäß einem der Ansprüche 1 bis 6, **dadurch gekennzeichnet, daß** der mindestens eine Füllstoff gemäß (e) eine BET-Oberfläche von bis zu 50 m²/g aufweist.

8. Silikonabformmasse gemäß einem der Ansprüche 1 bis 6, umfassend mindestens 2 voneinander verschiedene Füllstoffe gemäß (e), **dadurch gekennzeichnet, daß** mindestens ein Füllstoff eine BET-Oberfläche von bis zu 50 m²/g und mindestens ein Füllstoff eine BET-Oberfläche von mehr als 50 m²/g aufweist.

9. Silikonabformmasse gemäß einem der Ansprüche 1 bis 8, umfassend in der einen Komponente
(i) 15 bis 20 Gew.-% mindestens eines Organopolysiloxans mit mindestens zwei ungesättigten Gruppen im Molekül,
(ii) 1 bis 10 Gew.-% mindestens eines Organohydrogenpolysiloxans mit mindestens zwei SiH-Gruppen im Molekül,
(iii) 5 bis 8 Gew.-% mindestens eines Paraffinöls oder mindestens eines weißen Mineralöls oder einer Mischung aus mindestens einem Paraffinöl und mindestens einem weißen Mineralöl,
(iv) 60 bis 80 Gew.-% mindestens eines Füllstoffs,
jeweils bezogen auf das Gesamtgewicht dieser Komponente, wobei diese Komponente eine Brookfield-Viskosität im Bereich von 800 bis 2000 Pa*s aufweist, und in der anderen Komponente
(i') 5 bis 20 Gew.-% mindestens eines Organopolysiloxans mit mindestens zwei ungesättigten Gruppen im Molekül,
(ii') 0,00005 bis 0,05 Gew.-% mindestens eines Platin-Katalysators, berechnet als elementares Platin,
(iii') 0,5 bis 6 Gew.-% mindestens eines Paraffinöls oder mindestens eines weißen Mineralöls oder einer Mischung aus mindestens einem Paraffinöl und mindestens einem weißen Mineralöl,
(iv') 60 bis 80 Gew.-% mindestens eines Füllstoffs,
jeweils bezogen auf das Gesamtgewicht dieser Komponente,
wobei entweder der Bestandteil (a) oder der Bestandteil (i) oder der Bestandteil (i') oder sowohl der Bestandteil (i) als auch der Bestandteil (i') mindestens zwei voneinander verschiedene Organopolysiloxane mit mindestens zwei ungesättigten Gruppen im Molekül umfaßt, wobei
-- mindestens ein Organopolysiloxan eine Viskosität im Bereich von 25 bis 1000 mPa*s und
-- mindestens ein Organopolysiloxan eine Viskosität im Bereich von 60.000 bis 500.000 mPa*s
aufweist.

10. Sllikonabformmasse gemäß Anspruch 9, **dadurch gekennzeichnet, daß** Bestandteil (a) das mindestens eine Organopolysiloxan mit einer Viskosität im Bereich von 25 bis 1000 mPa*s in einem Anteil im Bereich von 1 bis 5 Gew.-% und das mindestens eine Organopolysiloxan mit einer Viskosität im Bereich von 60.000 bis 500.000 mPa*s in einem Anteil im Bereich von 10 bis 20 Gew.-% enthält.

11. Verfahren zum Anmischen einer Silikonabformmasse nach einem der Ansprüche 1 bis 10, **dadurch gekennzeichnet, daß** das Mischen der beiden Komponenten unter Verwendung eines automatischen motorbetriebenen Mischgerätes erfolgt.

12. Verfahren gemäß Anspruch 11, **dadurch gekennzeichnet, daß** es folgende Schritte umfaßt:
(A) Einbringen der in Primärverpackungen enthaltenen Komponenten in Sekundärverpackungen, die mit den Abmessungen des automatischen Mischgerätes kompatibel sind, oder
Einbringen der in Primärverpackungen enthaltenen Komponenten direkt in das automatische Mischgerät, oder
Einbringen der in einer Primärverpackung enthaltenen einen Komponente in eine Sekundärverpackung, die mit den entsprechenden Abmessungen des automatischen Mischgerätes kompatibel ist, und direktes Einbringen der in einer Primärverpackung enthaltenen Komponente in das automatische Mischgerät;
(B) Ausbringen der Komponenten aus dem automatischen Mischgerät über eine dynamische Mischkanüle unter Vermischung der Komponenten bei Ausdrückkräften von höchstens 4000 N.

13. Verwendung
- einer Mischung von mindestens zwei Organopolysiloxanen mit mindestens zwei ungesättigten Gruppen im Molekül, wobei
-- mindestens ein Organopolysiloxan eine Viskosität im Bereich von 25 bis 1000 mPa*s und
-- mindestens ein Organopolysiloxan eine Viskosität im Bereich von 60.000 bis 500.000 mPa*s,
aufweist, oder
- von sowohl dieser mindestens zwei Organopolysiloxane als auch mindestens eines Füllstoffs mit einer BET-Oberfläche von bis zu 50 m²/g,
als Bestandteile einer Silikonabformmasse oder mindestens einer der beiden Komponenten nach einem der Ansprüche 1 bis 10 zur Einstellung der rheologischen Eigenschaften der Silikonabformmasse oder der Komponenten.

14. Verwendung gemäß Anspruch 13, **dadurch gekennzeichnet, daß** die rheologischen Eigenschaften zu Ausdrückkräften in einem zum Ausbringen der Komponenten und der Silikonabformmasse verwendeten automatischen Mischgerät von höchstens 4000 N führen.

## Claims

1. Two-component addition-crosslinking silicone impression material which comprises, when the two components are in the mixed state,
(a) from 1 to 35% by weight of a mixture of at least two organopolysiloxanes having at least two unsaturated groups in the molecule, where at least one organopolysiloxane has a viscosity in the range of from 25 to 1000 mPa*s, and
at least one organopolysiloxane has a viscosity in the range of from 60,000 to 500,000 mPa*s,
(b) from 1 to 10% by weight of at least one organohydrogenpolysiloxane having at least two SiH groups in the molecule,
(c) from 0.00005 to 0.05% by weight of at least one platinum catalyst, calculated as elemental platinum,
(d) from 4 to 10% by weight of at least one liquid paraffin or at least one white mineral oil or of a mixture consisting of at least one liquid paraffin and at least one white mineral oil,
(e) from 50 to 90% by weight of at least one filler,
in each case based on the total weight of the silicone impression material, with one of the components exhibiting, prior to being mixed with the other component, a Brookfield viscosity in the range of from 800 to 2000 Pa*s, **characterized in that**, in the mixed state, the silicone impression material exhibits a consistency of less than or equal to 35 mm, as determined in accordance with ISO 4823.

2. Silicone impression material according to Claim 1 which comprises, when the components are in the mixed state,
(a) from 10 to 25% by weight of a mixture of at least two organopolysiloxanes having at least two unsaturated groups in the molecule, where at least one organopolysiloxane has a viscosity in the range of from 25 to 1000 mPa*s, and
at least one organopolysiloxane has a viscosity in the range of from 60,000 to 500,000 mPa*s,
(b) from 1 to 8% by weight of at least one organohydrogenpolysiloxane having at least two SiH groups in the molecule,
(c) from 0.0002 to 0.04% by weight of platinum catalyst, calculated as elemental platinum,
(d) from 5 to 8% by weight of at least one liquid paraffin or at least one white mineral oil or a mixture consisting of at least one liquid paraffin and at least one white mineral oil,
(e) from 65 to 83% by weight of at least one filler,
in each case based on the total weight of the silicone impression material.

3. Silicone impression material according to Claim 1 or 2, **characterized in that** the component which, prior to mixing with the other component, exhibits a Brookfield viscosity in the range of from 800 to 2000 Pa*s is used in a larger proportion by volume than the other component.

4. Silicone impression material according to one of Claims 1 to 3, **characterized in that** the two components are used in a volume ratio which is in the range of from 4:1 to 6:1.

5. Silicone impression material according to one of Claims 1 to 4, which additionally comprises at least one inhibitor, with a proportion in the range of from 0.00001 to 0.2% by weight, or at least one customary additive, with a proportion in the range of from 0.00001 to 2% by weight, or a mixture composed of at least one inhibitor and at least one customary additive, with a proportion of from 0.00002 to 2.2% by weight, in each case based on the total weight of the silicone impression material.

6. Silicone impression material according to one of Claims 1 to 5, **characterized in that** the at least one liquid paraffin or the at least one white mineral oil, or the mixture composed of at least one liquid paraffin and at least one white mineral oil according to (d) has a viscosity at 20°C which is in the range of from 100 to 400 mPa*s.

7. Silicone impression material according to one of Claims 1 to 6, **characterized in that** the at least one filler according to (e) has a BET surface area of up to 50 m²/g.

8. Silicone impression material according to one of Claims 1 to 6 which comprises at least 2 fillers according to (e) which differ from each other, **characterized in that** at least one filler has a BET surface area of up to 50 m²/g and at least one filler has a BET surface area of more than 50 m²/g.

9. Silicone impression material according to one of Claims 1 to 8, which comprises, in one of the components,
(i) from 15 to 20% by weight of at least one organopolysiloxane having at least two unsaturated groups in the molecule,
(ii) from 1 to 10% by weight of at least one organohydrogenpolysiloxane having at least two SiH groups in the molecule,
(iii) from 5 to 8% by weight of at least one liquid paraffin or at least one white mineral oil, or of a mixture consisting of at least one liquid paraffin and at least one white mineral oil,
(iv) from 60 to 80% by weight of at least one filler,
in each case based on the total weight of this component, with this component exhibiting a Brookfield viscosity in the range of from 800 to 2000 Pa*s, and, in the other component,
(i') from 5 to 20% by weight of at least one organopolysiloxane having at least two unsaturated groups in the molecule,
(ii') from 0.00005 to 0.05% by weight of at least one platinum catalyst, calculated as elemental platinum,
(iii') from 0.5 to 6% by weight of at least one liquid paraffin or at least one white mineral oil, or a mixture consisting of at least one liquid paraffin and at least one white mineral oil,
(iv') from 60 to 80% by weight of at least one filler,
in each case based on the total weight of this component,
where either the constituent (a) or the constituent (i) or the constituent (i'), or both the constituent (i) and the constituent (i') comprise(s) at least two organopolysiloxanes which differ from each other and which have at least two unsaturated groups in the molecule, where
- at least one organopolysiloxane has a viscosity in the range of from 25 to 1000 mPa*s, and
- at least one organopolysiloxane has a viscosity in the range of from 60,000 to 500,000 mPa*s.

10. Silicone impression material according to Claim 9, **characterized in that** constituent (a) contains the at least one organopolysiloxane, having a viscosity in the range of from 25 to 1000 mPa*s, in a proportion in the range of from 1 to 5% by weight and the at least one organopolysiloxane, having a viscosity in the range of from 60,000 to 500,000 mPa*s, in a proportion in the range of from 10 to 20% by weight.

11. Method for mixing a silicone impression material according to one of Claims 1 to 10, **characterized in that** the two components are mixed using an automatic motor-driven mixing unit.

12. Method according to Claim 11, **characterized in that** it comprises the following steps:
(A) introducing the components which are contained in primary packs into secondary packs which are compatible with the dimensions of the automatic mixing unit, or
introducing the components which are contained in primary packs directly into the automatic mixing unit, or
introducing one of the components which is contained in a primary pack into a secondary pack which is compatible with the corresponding dimensions of the automatic mixing unit and directly introducing the component contained in a primary pack into the automatic mixing unit;
(B) discharging the components from the automatic mixing unit by way of a dynamic small mixing tube, while mixing the components, at ejection forces of at most 4000 N.

13. Use
- of a mixture of at least two organopolysiloxanes having at least two unsaturated groups in the molecule, where
- at least one organopolysiloxane has a viscosity in the range of from 25 to 1000 mPa*s, and
- at least one organopolysiloxane has a viscosity in the range of from 60,000 to 500,000 mPa*s,
or
- of both these at least two organopolysiloxanes and at least one filler having a BET surface area of up to 50 m²/g
as constituents of a silicone impression material or of at least one of the two components as claimed in one of claims 1 to 10 for adjusting the rheological properties of the silicone impression material or components.

14. Use according to Claim 13, **characterized in that** the rheological properties lead to ejection forces of at most 4000 N in an automatic mixing unit which is used for discharging the components and the silicone impression material.

## Revendications

1. Matière pour empreinte à base de silicone à deux composants, réticulant par addition, contenant, à l'état mélangé des deux composants :
(a) 1 à 35 % en poids d'un mélange d'au moins deux organopolysiloxanes comportant au moins deux groupes insaturés dans la molécule,
au moins un organopolysiloxane présentant une viscosité de l'ordre de 25 à 1.000 mPa*s et
au moins un organopolysiloxane présentant une viscosité de l'ordre de 60.000 à 500.000 mPa*s,
(b) 1 à 10 % en poids d'au moins un organohydrogènepolysiloxane comportant au moins deux groupes SiH dans la molécule,
(c) 0,00005 à 0,05 % en poids d'au moins un catalyseur au platine, calculé sous forme de platine élémentaire,
(d) 4 à 10 % en poids d'au moins une huile de paraffine ou d'au moins une huile minérale blanche ou d'un mélange d'au moins une huile de paraffine et d'au moins une huile minérale blanche,
(e) 50 à 90 % en poids d'au moins un agent de charge,
chaque fois par rapport au poids total de la matière pour empreinte à base de silicone, un des composants présentant avant le mélange avec le premier composant une viscosité Brookfield de l'ordre de 800 à 2.000 Pa*s, **caractérisée en ce que** la matière pour empreinte à base de silicone présente à l'état mélangé une consistance déterminée conformément à l'ISO 4823 et inférieure ou égale à 35 mm.

2. Matière pour empreinte à base de silicone selon la revendication 1, comportant, à l'état mélangé des composants :
(a) 10 à 25 % en poids d'un mélange d'au moins deux organopolysiloxanes comportant au moins deux groupes insaturés dans la molécule,
au moins un organopolysiloxane présentant une viscosité de l'ordre de 25 à 1.000 mPa*s et
au moins un organopolysiloxane présentant une viscosité de l'ordre de 60.000 à 500.000 mPa*s,
(b) 1 à 8 % en poids d'au moins un organohydrogènepolysiloxane comportant au moins deux groupes SiH dans la molécule,
(c) 0,00002 à 0,04 % en poids de catalyseur au platine, calculé sous forme de platine élémentaire,
(d) 5 à 8 % en poids d'au moins une huile de paraffine ou d'au moins une huile minérale blanche ou d'un mélange d'au moins une huile de paraffine et d'au moins une huile minérale blanche,
(e) 65 à 83 % en poids d'au moins un agent de charge,
chaque fois par rapport au poids total de la matière pour empreinte à base de silicone.

3. Matière pour empreinte à base de silicone selon la revendication 1 ou 2, **caractérisé en ce que** le composant qui présente, avant mélange avec l'autre composant, une viscosité Brookfield de l'ordre de 800 à 2.000 mPa*s est utilisé en une proportion volumique plus grande que l'autre composant.

4. Matière pour empreinte à base de silicone selon l'une quelconque des revendications 1 à 3, **caractérisé en ce que** les deux composants sont utilisés en une proportion volumique de l'ordre de 4 : 1 à 6 : 1.

5. Matière pour empreinte à base de silicone selon l'une quelconque des revendications 1 à 4, comportant en plus au moins un inhibiteur dans une proportion de l'ordre de 0,00001 à 0,2 % en poids ou au moins un additif usuel dans une proportion de l'ordre de 0,00001 à 2 % en poids ou un mélange d'au moins un inhibiteur et d'au moins un additif usuel dans une proportion de 0,00002 à 2,2 % en poids par rapport au poids total de la matière pour empreinte à base de silicone.

6. Matière pour empreinte à base de silicone selon l'une quelconque des revendications 1 à 5, **caractérisée en ce que** l'au moins une huile de paraffine ou l'au moins une huile minérale blanche ou le mélange de l'au moins une huile de paraffine et de l'au moins une huile minérale blanche suivant (d) présente à 20° C une viscosité de l'ordre de 100 à 400 mPa*s.

7. Matière pour empreinte à base de silicone selon l'une quelconque des revendications 1 à 6, **caractérisée en ce que** l'au moins un agent de charge suivant (e) présente une surface BET allant jusqu'à 50 m²/g.

8. Matière pour empreinte à base de silicone selon l'une quelconque des revendications 1 à 6, comprenant au moins 2 agents de charge suivant (e) différents l'un de l'autre, **caractérisée en ce qu'**au moins un agent de charge présente une surface BET allant jusqu'à 50 m²/g et qu'au moins un agent de charge présente une surface BET supérieure à 50 m²/g

9. Matière pour empreinte à base de silicone selon l'une quelconque des revendications 1 à 8, comportant dans un composant
(i) 15 à 20 % en poids d'au moins un organopolysiloxane comprenant au moins deux groupes insaturés dans la molécule,
(ii) 1 à 10 % en poids d'au moins un organohydrogènepolysiloxane comprenant au moins deux groupes SiH dans la molécule,
(iii)5 à 8 % en poids d'au moins une huile de paraffine ou d'au moins une huile minérale blanche ou d'un mélange d'au moins une huile de paraffine et d'au moins une huile minérale blanche,
(iv) 60 à 80 % en poids d'au moins un agent de charge,
à chaque fois par rapport au poids total de ce composant, ce composant présentant une viscosité Brookfield de l'ordre de 800 à 2.000 mPa*s, et, dans l'autre composant,
(i') 5 à 20 % en poids d'au moins un organopolysiloxane comprenant au moins deux groupes insaturés dans la molécule,
(ii') 0,00005 à 0,05 % en poids d'au moins un catalyseur au platine, calculé sous forme de platine élémentaire,
(iii') 0,5 à 6 % en poids d'au moins une huile de paraffine ou d'au moins une huile minérale blanche ou d'un mélange d'au moins une huile de paraffine et d'au moins une huile minérale blanche,
(iv')60 à 80 % en poids d'au moins un agent de charge,
à chaque fois par rapport au poids total de ce composant, tandis que soit la composante (a), soit la composante (i), soit la composante (i'), soit aussi bien la composante (i) que la composante (i') comprend au moins deux organopolysiloxanes différents l'un de l'autre et contenant au moins deux groupes insaturés dans la molécule,
- au moins un organopolysiloxane présentant une viscosité de l'ordre de 25 à 1.000 mPa*s et
- au moins un organopolysiloxane présentant une viscosité de l'ordre de 60.000 à 500.000 mPa*s.

10. Matière pour empreinte à base de silicone selon la revendication 9, **caractérisée en ce que** la composante (a) contient l'au moins un organopolysiloxane d'une viscosité de l'ordre de 25 à 1.000 mPa*s dans une proportion de l'ordre de 1 à 5 % en poids et l'au moins un organopolysiloxane d'une viscosité de l'ordre de 60.000 à 500.000 mPa*s dans une proportion de l'ordre de 10 à 20 % en poids.

11. Procédé de mélange d'une matière pour empreinte à base de silicone selon l'une quelconque des revendications 1 à 10, **caractérisé en ce que** le mélange des deux composants est réalisé en utilisant un appareil mélangeur motorisé automatique.

12. Procédé selon la revendication 11, **caractérisé en ce qu'**il comprend les étapes suivantes :
(A) introduction des composants contenus dans des emballages primaires dans des emballages secondaires qui sont compatibles avec les dimensions de l'appareil mélangeur automatique ou
introduction des composants contenus dans des emballages primaires directement dans l'appareil mélangeur automatique ou introduction du composant contenu dans un emballage primaire dans un emballage secondaire qui est compatible avec les dimensions correspondantes de l'appareil mélangeur automatique et introduction directe du composant contenu dans un emballage primaire dans l'appareil mélangeur automatique ;
(B) expulsion des composants hors de l'appareil mélangeur automatique par une canule de mélange dynamique tout en mélangeant les composants sous des forces d'expulsion de 4.000 N au plus.

13. Utilisation
- d'un mélange d'au moins deux organopolysiloxanes comportant au moins deux groupes insaturés dans la molécule,
- au moins un organopolysiloxane présentant une viscosité de l'ordre de 25 à 1.000 mPa*s et
- au moins un organopolysiloxane présentant une viscosité de l'ordre de 60.000 à 500.000 mPa*s
ou
- d'aussi bien ces au moins deux organopolysiloxanes que de cet au moins un agent de charge ayant une surface BET allant jusqu'à 50 m²/g
comme composantes d'une matière pour empreinte à base de silicone selon l'une quelconque des revendications 1 à 10 pour ajuster les propriétés rhéologiques de la matière pour empreinte à base de silicone ou des composants.

14. Utilisation selon la revendication 13, **caractérisée en ce que** les propriétés rhéologiques entraînent des forces d'expulsion de 4.000 N au plus dans un appareil mélangeur automatique utilisé pour expulser les composants et la matière pour empreinte à base de silicone.
